# FASCICULE DE BREVET EUROPEEN

(11) **EP 1 558 172 B1**
(45) Date de publication et mention de la délivrance du brevet: **20.02.2008**
(21) Numéro de dépôt: 03767882.8
(22) Date de dépôt: 03.11.2003
(51) Int. Cl.: A61F 2/00

(54) **PIECE COMPOSITE INTERMEDIAIRE POUR FACONNAGE DE PROTHESES DE RENFORT**
ZUSAMMENGESETZTES ZWISCHENSTÜCK ZUR HERSTELLUNG EINER PROTHESE
INTERMEDIATE COMPOSITE PART FOR SHAPING REINFORCING PROSTHESES

(30) Priorité: 04.11.2002 FR 0213964
(43) Date de publication de la demande: 03.08.2005
(73) Titulaire: SOFRADIM PRODUCTION, 01600 Trévoux (FR)
(72) Inventeur: THERIN, Michel, F-69004 LYON (FR); GRAVAGNA, Philippe, F-69450 Irigny (FR)
(74) Mandataire: Brédeville, Odile Marie
(86) Numéro de dépôt international: PCT/FR2003/003270
(87) Numéro de publication internationale: WO 2004/041123

(56) Documents cités:
- EP-A- 0 895 762
- FR-A- 2 724 563
- US-A- 4 603 695
- US-B1- 6 451 032

## Description

La présente invention concerne une pièce composite intermédiaire pour le façonnage de prothèses de renfort de structures tissulaires.

Les prothèses de renfort servent de manière générale à soutenir les tissus et peuvent être utilisées pour différents endroits du corps. Ces prothèses de renfort peuvent être prévues pour une implantation temporaire. Dans ce cas, elles sont constituées d'un matériau résorbable. Elles peuvent au contraire être prévues pour une implantation définitive et sont alors constituées par exemple d'un support textile en un matériau non résorbable.

Les supports textiles sont intrinsèquement adhésiogènes et fibrosants quelle que soit la nature des tissus avec lesquels ils sont mis en contact. Cette propriété exprimée vis à vis des tissus de soutien (muscles, aponévroses, fascias etc..) constitue même un prérequis indispensable pour la qualité du résultat. En revanche, vis à vis d'autres structures plus fragiles, la présence d'un support textile lors de l'inflammation cicatricielle initiale favorise l'établissement de liens fibreux denses, là où n'existaient que des liens lâches tels que ceux que procurent les tissus conjonctifs interstitiels pour les organes extrapéritonéaux et où n'existait aucun lien pour les organes intrapéritonéaux. De ce fait, la nature poreuse des supports textiles est souvent à l'origine du développement d'adhérences et d'érosions post chirurgicales.

Une façon de remédier à ce problème est de rendre lisse la face de la prothèse sur laquelle on ne souhaite pas la formation de ces adhérences ou de ces érosions, de préférence en la recouvrant par un matériau résorbable.

Par exemple, en chirurgie pariétale, on cherche à réparer, par l'implantation de prothèses de renforts, les parois de la cavité abdominale qui ont été endommagées par des hernies ou des éventrations. Si elle est implantée dans la cavité abdominale en situation intra-péritonéale, la prothèse de renfort doit donc présenter de préférence une face propice à la colonisation cellulaire côté paroi pour une reconstruction chirurgicale efficace et une face lisse peu favorable à la formation d'adhérences post-chirurgicales côté viscères. Dans un tel cas, les prothèses de renforts se présentent de préférence sous la forme d'un textile poreux dont une face est recouverte superficiellement par une couche ou membrane résorbable pour éviter la formation d'adhérences post-chirurgicales, l'autre face étant laissée libre pour une intégration tissulaire intime et précoce de la paroi à soutenir. WO99/06080 décrit de telles prothèses.

Un autre exemple est le cas des traitements chirurgicaux pour le soutien de tissus ou la réparation des prolapsus. Les tissus concernés par ces traitements, tissus dits extrapéritonéaux, sont particulièrement exposés car pouvant rentrer au contact des deux faces de la prothèse. Ces tissus sont par exemple des viscères creux, comme la vessie, le vagin, l'utérus ou le rectum, ou des canaux naturels de l'organisme, comme l'urètre. L'oesophage, la trachée, les vaisseaux sanguins, les nerfs, les tendons, la dure mère constituent d'autres exemples de structures fragiles qu'il faudrait protéger en cas d'utilisation de renforts poreux à proximité. Pour de tels tissus dits fragiles, les liens fibreux postérieurs à l'inflammation cicatricielle initiale peuvent être aggravés d'érosion ou de fistules. Dans ce cas, il est nécessaire, pendant la phase initiale de cicatrisation, de protéger ces tissus fragiles environnants en recouvrant les deux faces de la prothèse par un matériau lisse.

Les traitements chirurgicaux pour le soutien de tissus ou la réparation des prolapsus impliquent de soutenir ces tissus dits fragiles (vessie, vagin, utérus, rectum) tout en les reliant à des tissus plus résistants situés dans des zones anatomiquement stables. Ainsi, la partie de la prothèse en contact avec les tissus fragiles doit présenter des faces lisses tandis que les parties de la prothèse en contact avec les tissus éloignés résistants sont de préférence poreuses pour permettre un meilleur ancrage de la prothèse. A cet égard, l'état de la technique antérieure décrit une prothèse adaptée au traitement de l'incontinence chez la femme, dont la partie centrale est revêtue de silicone hydrophobe non résorbable et dont les pièces latérales d'ancrage sont des treillis tricotés ou non tissés. Toutefois, une telle prothèse est peu souhaitable dans la protection des structures fragiles en raison de la nature hydrophobe et non résorbable du silicone.

Par ailleurs, il peut être souhaitable, pour des raisons d'hygiène ou de facilitation d'utilisation par le chirurgien de recouvrir seulement une partie d'une prothèse. Par exemple, on peut vouloir rigidifier temporairement une partie d'une prothèse en la recouvrant d'un film continu pour une utilisation en chirurgie laparoscopique.

Enfin, les prothèses de renforts doivent également être adaptées à la taille et la morphologie interne du patient.

Ainsi, il apparaît qu'il existe autant, de formes et de natures de prothèse que de pathologies et de patients. Or, il serait fastidieux et dangereux de fabriquer une prothèse de bout en bout sur le site opératoire. Cela prendrait énormément de temps, rallongerait le temps d'anesthésie nécessaire à l'opération et augmenterait les risques de complication.

Il serait par conséquent intéressant que le chirurgien chargé d'opérer une hernie ou un prolapsus puisse disposer d'une pièce composite intermédiaire dans laquelle il pourrait découper, de manière simple et rapide, une prothèse de renfort adaptée à la morphologie et à la pathologie du patient qu'il est en train d'opérer.

La présente invention a donc pour objet une pièce composite intermédiaire, adaptée pour le façonnage d'une prothèse composite de renfort, caractérisée en ce qu'elle comprend :
- i) un support textile poreux comprenant un arrangement de fils composés chacun d'au moins un brin en matériau polymère non résorbable, ledit support textile déterminant le ou les bords libres de la pièce, et
- ii) un matériau résorbable hydrophile revêtant ledit support textile, au moins d'un côté de ce dernier, s'étendant en surface selon une zone dite protégée et ménageant de part et d'autre de cette dernière, deux zones dites non protégées du support textile, limitées par lesdits bords libres et exemptes de tout dit matériau résorbable.

La présente invention a également pour objet l'utilisation d'une pièce composite intermédiaire telle que ci-dessus pour obtenir une prothèse composite de renfort.

La présente invention a encore pour objet un procédé d'obtention d'une prothèse composite de renfort, caractérisé en ce que :
- i) on dispose d'une pièce composite intermédiaire telle que décrite ci-dessus,
- ii) on découpe la pièce composite selon une ligne de découpe déterminant dans cette dernière au moins deux zones d'accroche tissulaire, de part et d'autre d'une zone d'intégration tissulaire, les zones d'accroche étant découpées dans les deux zones non protégées respectivement, et la zone d'intégration étant découpée dans la zone protégée.

Grâce à l'invention, le chirurgien peut découper, sur le site opératoire la prothèse la mieux adaptée à la morphologie de son patient compte tenu de la pathologie pour laquelle il est en train de l'opérer.

Par support "textile poreux", on entend au sens de la présente invention, un support textile présentant des vides sous la forme d'interstices et/ou de volumes. Plus précisément, le support textile poreux est composé d'un arrangement de fils définissant une texture microporeuse et/ou une texture macroporeuse.

L'arrangement de fils dont il est question est un enchevêtrement ordonné, par exemple tissé selon toute armure appropriée, ou tricoté, ou encore désordonné, par exemple non tissé. Dans une forme privilégiée de réalisation de l'invention, l'arrangement de fils constitue une structure tricotée. Chaque fil dont il s'agit comprend au moins un brin ou filament, continu ou discontinu, en matériau polymère non résorbable; chaque fil peut comprendre d'autres fils ou filaments par exemple en matériau polymère résorbable.

Par "matériau polymère", on entend tout matériau, sous forme singulière ou d'alliage, comprenant un polymère synthétique ou naturel, obtenu par exemple par polymérisation ou copolymérisation. Le matériau polymère non résorbable selon l'invention peut être un polypropylène ou encore un polyester de type polyéthylène terephtalate.

La texture microporeuse comprend au moins les interstices compris entre au moins deux fils aux endroits de contact d'un fil avec au moins un autre fil. Dans le cas où au moins un fil comprend plusieurs brins assemblés ou non, la texture microporeuse comprend en outre les interstices entre brins d'un même fil.

On peut donc définir la microporosité du support textile comme étant la somme i°) le cas échéant des interstices compris entre au moins deux brins au sein d'un même fil et ii°) des interstices compris entre au moins deux fils aux endroits de contact d'un fil avec au moins un autre fil.

La texture macroporeuse ou macroporosité comprend les volumes dont la surface S est définie par les espaces vides entre au moins deux fils, en dehors de leurs endroits de contact, et dont la hauteur H est définie par l'épaisseur du support textile. Selon la présente invention, on considère que le support textile est plat lorsqu'il constitue une structure bidimensionnelle, de préférence lorsque son épaisseur est inférieure ou égale à 5 fois le diamètre moyen des fils le constituant. Dans ce cas, on parlera de macroporosité bidimensionnelle ou de texture macroporeuse bidimensionnelle. Dans le cas où le support textile constitue une structure tridimensionnelle, de préférence lorsque l'épaisseur du support textile est strictement supérieure à 5 fois le diamètre moyen des fils constituant le support, on parlera de macroporosité tridimensionnelle ou de texture macroporeuse tridimensionnelle.

Dans une forme préférée de réalisation de l'invention, le support textile constitue une structure bidimensionnelle.

Le support textile est constitué d'un matériau polymère non résorbable communément utilisé en chirurgie. De préférence, ce matériau polymère non résorbable est choisi dans le groupe comprenant les polypropylènes, les polyesters comme les polyéthylène terephtalates, les polyamides et/ou leurs mélanges. Dans une forme privilégiée de réalisation de l'invention, ce matériau polymère est du polypropylène. Comme support textile à base de polypropylène convenant à la présente invention, on peut citer le produit vendu sous la dénomination commerciale « Parietene®» par la société Sofradim, le produit vendu sous la dénomination commerciale « Prolene® » par la société Ethicon ou encore le produit vendu sous la dénomination commerciale « Marlex® » par la société Bard.

Le matériau résorbable hydrophile selon l'invention est choisi dans le groupe constitué par les collagènes, les polysaccharides et leurs mélanges. Parmi les collagènes utilisables selon l'invention, on peut citer :
1) du collagène dont la structure hélicoïdale est au moins partiellement thermo-dénaturée, sans dégradation hydrolytique, dont la méthode de préparation est décrite dans WO99/06080,
2) du collagène natif, non chauffé, filmé, avec ou sans glycérine, réticulé par irradiation gamma ou par d'autres moyens chimiques ou physiques,
3) et/ou leurs mélanges.

Parmi les polysaccharides utilisables comme matériau hydrophile résorbable selon l'invention, on peut citer la cellulose oxydée, l'acide hyaluronique, l'amidon, le chitosan, les dextranes réticulés et/ou leurs mélanges. Tous ces matériaux sont bien connus de l'homme de l'art. Comme cellulose oxydée convenant à la présente invention, on peut citer le produit vendu sous la dénomination commerciale « Interceed® » par la société Ethicon. Comme acide hyaluronique convenant à la présente invention, on peut citer le produit vendu sous la dénomination commerciale « Hyalobarrier® » par la société Fidia Advanced Biopolymers, ou le produit vendu sous la dénomination commerciale « Seprafilm® » par la société Genzyme.

Le matériau résorbable hydrophile revêt le support textile sur un de ces côtés ou sur ses deux côtés. Selon l'invention, on définit par « zone protégée » la zone du support textile revêtue par le matériau résorbable hydrophile.

Le matériau résorbable peut revêtir le support textile au moyen d'une membrane liée au support textile par collage et/ou imprégnation partielle ou encore au moyen d'une enduction obturant la microporosité. L'enduction peut éventuellement obturer en outre la macroporosité du support textile sur une zone dite zone obturée.

Dans le cas où le matériau résorbable revêt le support textile au moyen d'une membrane, cette dernière est de préférence liée au moins superficiellement au support textile, de préférence encore sur une certaine épaisseur par absorption capillaire des fils constituant le support textile. La membrane de matériau résorbable est de préférence continue, lisse et non poreuse. Elle peut former une couche plane d'une épaisseur pouvant aller jusqu'à plusieurs millimètres.

La pièce composite selon l'invention peut être obtenue en appliquant sur la partie du côté du support textile à recouvrir une couche de solution de matériau résorbable qui formera la membrane après gélification et séchage. Une autre façon de procéder est de préparer une première couche de solution de matériau résorbable dans un plateau. Après gélification de cette première couche par refroidissement, on applique à sa surface une deuxième couche à partir de la même solution. On applique ensuite la partie du côté du support textile à recouvrir sur la deuxième couche, avant gélification, de telle sorte que l'ancrage s'effectue pendant le séchage de cette deuxième couche. On obtient ainsi une membrane plane présentant une épaisseur pouvant aller jusqu'à plusieurs millimètres. Si l'on souhaite que les deux côtés du support textile soient recouverts, on recommence la même opération sur l'autre côté du support.

La pièce composite intermédiaire peut aussi être préparée par pulvérisation sur tout ou partie du support textile, de préférence à l'aide d'un pochoir ou d'un masque de protection, de la solution de matériau résorbable hydrophile. La pulvérisation peut être pratiquée sur une première face du textile. Après 15 minutes à 2 heures de maturation du gel formé à la surface du textile, on peut pulvériser la solution sur l'autre face. Après la deuxième pulvérisation, on laisse sécher l'ensemble sous flux d'air stérile. Après maturation, on stérilise le tout par de l'oxyde d'éthylène.

Dans le cas où le revêtement est une enduction, le matériau résorbable hydrophile enduit la zone protégée du support textile en formant un film enveloppant et pénétrant dans l'arrangement de fils, qui obture la texture microporeuse, autrement dit au moins la microporosité du support, mais sans former une couche ou membrane épaisse revêtant au moins une face du support textile. Le film enrobe directement ou indirectement au moins chaque fil complètement, il réalise une enduction. De plus, dans le cas où chaque fil du support est constitué d'un seul brin, le film résorbable remplit et donc obture tous les interstices compris entre au moins deux fils aux endroits de contact d'un fil avec au moins un autre fil. Dans le cas où au moins un fil comporte plusieurs brins, assemblés ou non les uns aux autres, alors le film remplit et donc obture également tous les interstices entre brins d'un même fil.

Dans une forme préférée de réalisation de l'invention, le film qui enduit le support textile comme décrit dans le paragraphe ci-dessus est discontinu et préserve la texture macroporeuse du support textile. Le film enrobe alors chaque fil et laisse libres les volumes définissant la macroporosité. Ainsi, le renfort conserve une macroporosité prononcée pour un ancrage mécanique rapide et une réhabitation cellulaire immédiate. Une telle enduction par un film discontinu permet également de rigidifier temporairement le renfort et facilite sa manipulation par le chirurgien. Cette dernière propriété est particulièrement avantageuse en chirurgie laparoscopique.

De préférence, le film en matériau résorbable présente une épaisseur inférieure ou égale à 500 microns, et de préférence encore, allant de 10 à 100 microns.

Dans une autre forme de réalisation de l'invention, le film peut également obturer la macroporosité du support textile sur une partie de la zone protégée. Selon l'invention, on définit par « zone obturée » la partie de la zone protégée pour laquelle la macroposité est obturée. Sur cette zone obturée, le film remplit et obture les volumes bidimensionnels ou tridimensionnels définissant la macroporosité. Il est donc continu.

Lorsque le revêtement est une enduction, la pièce composite intermédiaire selon l'invention peut être préparée selon le procédé comprenant les étapes suivantes :
- i) préparation d'une solution d'un matériau résorbable hydrophile, à l'état fluide ou liquide,
- ii) immersion dans cette solution de la partie du support textile à recouvrir,
- iii) retrait de la partie du support textile imprégnée de solution et séchage de cette dernière.

La solution de matériau résorbable hydrophile doit être suffisamment fluide pour qu'elle imprègne le support textile jusqu'à obturation de la microporosité. Cette solution peut être chauffée à une température inférieure à 50°C. De préférence, afin de faciliter cette étape d'imprégnation, la solution A présente une viscosité inférieure ou égale à 1000 centipoises, et de préférence encore allant de 50 à 200 centipoises, cette viscosité étant mesurée à l'aide d'un viscosimètre CONTRAVES -TV par exemple à la température choisie inférieure 50°C. Les parties du support textile qui ne sont pas destinées à être enduites peuvent être recouvertes d'une membrane protectrice durant l'étape d'immersion. Dans le cas où la zone enduite représente une bande centrale du support textile, on peut aussi préalablement plier le support en forme de U, la partie à enduire se trouvant être la barre horizontale du U, et tremper ainsi la partie à enduire dans la solution de matériau résorbable tout en préservant les parties latérales (barres verticales du U).

Dans une forme préférée de réalisation de l'invention, la pièce composite intermédiaire se présente sous la forme d'une pièce rectangulaire et la zone protégée, respectivement obturée, représente une bande centrale de cette pièce.

Grâce à l'invention, le praticien peut librement façonner des prothèses de renfort en découpant dans la pièce composite intermédiaire des bandes de géométrie adaptée, et les disposer de façon à procurer un soutien non agressif, grâce à la zone protégée, respectivement obturée, vis-à-vis des structures tissulaires fragiles, et un ancrage rapide dans les zones non protégées à distance des structures fragiles.

De telles prothèses présentent des zones à accroche immédiate pour une suspension efficace dès l'implantation, par rétraction des tissus conjonctifs interstitiels, des fascias et des aponévroses au niveau des bords libres des fils, des zones relativement fibrosantes pour un ancrage mécanique durable au niveau de la partie non protégée du support textile, et des zones à intégration non agressive et peu fibrosante au niveau de la zone centrale protégée, respectivement obturée, du renfort.

Une prothèse de renfort peut être façonnée à partir de la pièce composite intermédiaire selon l'invention selon le procédé suivant :
- i) on dispose d'une pièce composite intermédiaire telle que décrite ci-dessus,
- ii) on découpe la pièce composite selon une ligne de découpe déterminant dans cette dernière au moins deux zones d'accroche tissulaire, de part et d'autre d'une zone de soutien tissulaire, les zones d'accroche étant découpées dans les deux zones non protégées respectivement, et la zone de soutien étant découpée dans la zone protégée.

Dans une forme préférée de réalisation de l'invention, la ligne de découpe détermine une bandelette à bords parallèles droits. Dans une autre forme de réalisation de l'invention, la ligne de découpe détermine une bandelette à bords parallèles incurvés en arche. Dans une autre forme de réalisation de l'invention, la ligne de découpe détermine une bandelette à bords non parallèles, renflée en région centrale, pour soutenir sur une grande surface les structures prolabées et effilée aux extrémités pour constituer des bandelettes de suspension d'ancrage. Ces bandelettes sont de largeur et longueur modulables.

La présente invention sera mieux comprise en se référant aux figures suivantes :
- la figure 1 est une vue de dessus représentant une pièce composite intermédiaire selon l'invention, de forme rectangulaire et dont la zone protégée a la forme d'une bande centrale
- la figure 2 est de dessus représentant une pièce composite intermédiaire selon l'invention sur laquelle on fait apparaître les formes de prothèses que l'on peut découper.

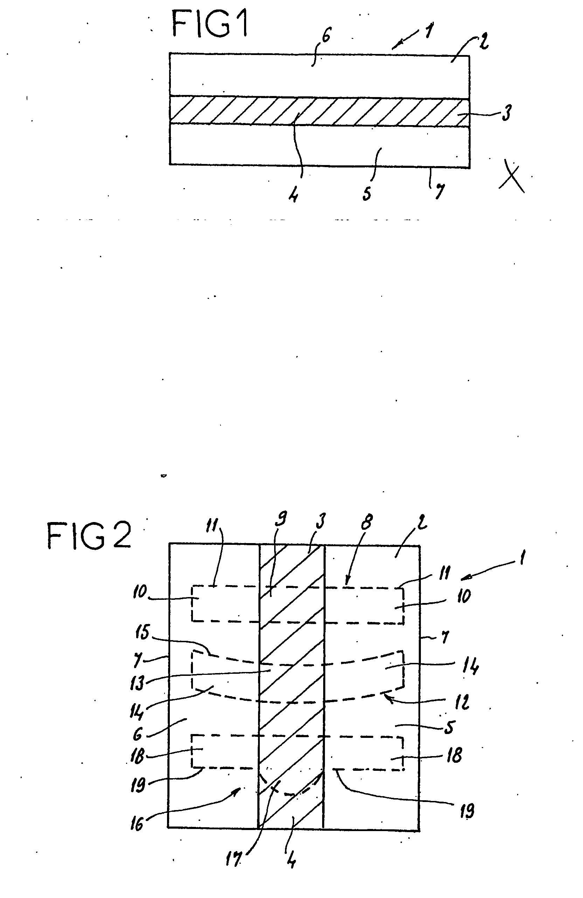

En se référant à la Figure 1, une pièce 1 composite intermédiaire de forme rectangulaire est représentée qui comprend un support textile 2 dont la partie centrale 4, ici selon la forme d'une bande centrale, est protégée par un matériau résorbable 3. Ce matériau résorbable peut revêtir la partie 4 selon un film ou selon une membrane. Les parties 5 et 6 du support textile 2 limitées par leurs bords libres 7 sont exemptes de matériau résorbable.

La figure 2 montre une pièce selon la figure 1 sur laquelle on a fait figurer les découpes selon lesquelles on peut découper des prothèses. Ainsi, il est possible de découper une bandelette 8 à bords parallèles qui présente une partie centrale 9 protégée et deux parties latérales 10 exemptes de matériau résorbable. Une fois découpée, la bandelette 8 constitue une prothèse composite de renfort dont les parties latérales 10 définissent des zones d'accroche tissulaire relativement fibrosantes pour un ancrage mécanique durable tandis que la partie centrale 9 définit une zone de soutien tissulaire non agressive et peu fibrosante. Les bords libres 11 définissent une zone à accroche immédiate pour une suspension efficace dès l'implantation. Il est également possible de découper dans la pièce 1 une bandelette 12 à bords parallèles incurvés en arche qui présente une partie centrale 13 protégée et deux parties latérales 14 exemptes de matériau résorbable. Une fois découpée, la bandelette 12 constitue une prothèse composite de renfort dont les parties latérales 14 définissent des zones d'accroche tissulaire relativement fibrosantes tandis que la partie centrale 13 définit une zone de soutien tissulaire non agressive et peu fibrosante. Les bords libres 15 définissent une zone à accroche immédiate. Il est également possible de découper dans la pièce 1 une bandelette 16 à bords non parallèles qui présente une partie centrale 17 protégée renflée et deux parties latérales 18 effilées et exemptes de matériau résorbable. Une fois découpée, la bandelette 16 constitue une prothèse composite de renfort dont les parties latérales 18 effilées définissent des zones d'accroche tissulaire relativement fibrosantes tandis que la partie centrale 17 renflée définit une zone de soutien tissulaire non agressive et peu fibrosante. Les bords libres 19 définissent une zone à accroche immédiate.

### EXEMPLE 1:

On réalise un support textile de forme rectangulaire de dimensions 25 X 20 cm².

On prépare une solution comprenant du collagène modifié par coupure oxydative et chauffage, du polyéthylène glycol et de la glycérine. Les concentrations pondérales en collagène, en polyéthylène glycol et en glycérine sont de préférence respectivement comprises entre environ 2 et 9% pour le collagène, environ 0,6 et 3% pour le polyéthylène glycol, environ 0,3 et 1,2% pour la glycérine.

Cette solution présente une viscosité allant de 50 à 200 centipoises. On immerge une bande centrale de largeur 6 cm du support textile dans cette solution, puis on le retire et on le laisse gélifier pendant 30 minutes. On répète cette opération autant de fois qu'il est nécessaire pour obtenir un film de densité 0.133 g/cm².

On obtient une pièce composite intermédiaire dont la bande centrale de largeur 6 cm est une zone obturée selon l'invention et dont les parties latérales sont exemptes de matériau résorbable.

On découpe au sein de cette pièce dans le sens de la largeur une bandelette à bords parallèles de dimensions 20 X 4 cm² et on obtient une prothèse composite de renfort dont la partie centrale de dimensions 6 X 4 cm² est une partie obturée et les parties latérales, chacune de dimensions 7 X 4 cm² sont des parties non protégées relativement fibrosantes

### EXEMPLE 2 :

A partir du même support textile et de la même solution que décrits dans l'exemple 1, on pulvérise la solution sur la bande centrale du support textile qu'on souhaite protéger. La pulvérisation est pratiquée sur une face du textile. Après 15 minutes à 2 heures de maturation du gel formé à la surface du textile, on pulvérise la solution sur l'autre face. Après la deuxième pulvérisation, on laisse sécher l'ensemble sous flux d'air stérile. On pulvérise la quantité de solution nécessaire pour obtenir un film de densité finale 0.133 g/cm². Après maturation, on stérilise le tout par de l'oxyde d'éthylène.

On obtient une pièce composite intermédiaire dont la bande centrale de largeur 6 cm est une zone obturée selon l'invention et dont les parties latérales sont exemptes de matériau résorbable.

On découpe au sein de cette pièce dans le sens de la largeur une bandelette à bords parallèles de dimensions 20 X 4 cm² et on obtient une prothèse composite de renfort dont la partie centrale de dimensions 6 X 4 cm² est une partie obturée et les parties latérales, chacune de dimensions 7 X 4 cm² sont des parties non protégées relativement fibrosantes.

## Revendications

1. Pièce (1) composite intermédiaire, adaptée pour le façonnage d'une prothèse composite de renfort, **caractérisée en ce qu'**elle comprend :
- i) un support textile (2) poreux comprenant un arrangement de fils composés chacun d'au moins un brin en matériau polymère non résorbable, ledit support textile déterminant le ou les bords libres (7) de la pièce (1), et
- ii) un matériau résorbable hydrophile (3) revêtant ledit support textile, au moins d'un côté de ce dernier, s'étendant en surface selon une zone dite protégée (4) et ménageant de part et d'autre de cette dernière, deux zones dites non protégées (5, 6) du support textile, limitées par lesdits bords libres et exemptes de tout dit matériau résorbable.

2. Pièce selon la revendication 1, **caractérisée en ce que** l'arrangement de fils constitue une structure tricotée.

3. Pièce selon la revendication 1 ou 2, **caractérisée en ce que** le support textile (2) constitue une structure bidimensionnelle.

4. Pièce selon l'une quelconque des revendications précédentes, **caractérisée en ce que** le matériau polymère non résorbable est choisi dans le groupe comprenant les polypropylènes, les polyesters, les polyamides et/ou leurs mélanges.

5. Pièce selon l'une quelconque des revendications précédentes, **caractérisée en ce que** le matériau résorbable (3) est choisi dans le groupe constitué par les collagènes, les polysaccharides et leurs mélanges.

6. Pièce selon l'une quelconque des revendications précédentes, **caractérisée en ce que** le matériau résorbable (3) revêt le support textile (2) sur ses deux côtés.

7. Pièce selon la revendication 1 ou 2, **caractérisée en ce que** le matériau résorbable (3) revêt le support textile (2) au moyen d'une membrane liée au support textile par collage et/ou imprégnation partielle.

8. Pièce selon la revendication 1 ou 2, **caractérisée en ce que** le support textile (2) définit une microporosité et une macroporosité.

9. Pièce selon la revendication précédente, **caractérisée en ce que** le matériau résorbable (3) revêt le support textile (2) au moyen d'une enduction obturant la microporosité du support textile.

10. Pièce selon la revendication précédente, **caractérisée en ce que** l'enduction obture également la macroporosité du support textile (2) sur une zone dite zone obturée.

11. Pièce selon l'une quelconque des revendications précédentes, **caractérisée en ce qu'**elle est rectangulaire et **en ce que** la zone protégée (4), représente une bande centrale de la pièce.

12. Pièce selon la revendication 10, **caractérisée en ce qu'**elle est rectangulaire et **en ce que** la zone obturée représente une bande centrale de la pièce.

13. Utilisation d'une pièce composite intermédiaire selon l'une quelconque des revendications 1 à 12, pour obtenir une prothèse composite de renfort.

14. Procédé d'obtention d'une prothèse composite de renfort, **caractérisé en ce que** :
- i) on dispose d'une pièce (1) composite intermédiaire selon l'une quelconque des revendications 1 à 12,
- ii) on découpe la pièce (1) composite selon une ligne de découpe déterminant dans cette dernière au moins deux zones d'accroche tissulaire (10, 14, 18), de part et d'autre d'une zone de soutien tissulaire (9, 13, 17), les zones d'accroche étant découpées dans les deux zones non protégées (5, 6) respectivement, et la zone de soutien tissulaire étant découpée dans la zone protégée (4).

15. Procédé selon la revendication 14, **caractérisé en ce que** la ligne de découpe détermine une bandelette à bords parallèles droits (8).

16. Procédé selon la revendication 14, **caractérisé en ce que** la ligne de découpe détermine une bandelette à bords parallèles incurvés en arche (12).

17. Procédé selon la revendication 14, **caractérisé en ce que** la ligne de découpe détermine une bandelette à bords non parallèles (16), renflée en région centrale et effilée aux extrémités.

## Claims

1. An intermediate composite part (1) designed for forming a composite reinforcement prosthesis and comprising:
- i) a porous textile support (2) which includes an arrangement of threads each composed of at least one filament of nonabsorbable polymer material, said textile support defining the free edge or edges (7) of the part (1), and
- ii) a hydrophilic absorbable material (3) covering said textile support, at least on one side of the latter, extending across the surface of what is called a protected zone (4) and creating, on each side of the latter, two unprotected zones (5, 6) of the textile support which are limited by said free edges and are devoid of any of said absorbable material.

2. The part as claimed in claim 1, wherein the arrangement of threads constitutes a knitted structure.

3. The part as claimed in claim 1 or 2, wherein the textile support (2) constitutes a two-dimensional structure.

4. The part as claimed in any one of the preceding claims, wherein the nonabsorbable polymer material is chosen from the group comprising polypropylenes, polyesters, polyamides and/or their mixtures.

5. The part as claimed in any one of the preceding claims, wherein the absorbable material (3) is chosen from the group formed by collagens, polysaccharides, and their mixtures.

6. The part as claimed in any one of the preceding claims, wherein the absorbable material (3) covers the textile support (2) on both of its sides.

7. The part as claimed in claim 1 or 2, wherein the absorbable material (3) covers the textile support (2) by means of a membrane linked to the textile support by bonding and/or partial impregnation.

8. The part as claimed in claim 1 or 2, wherein the textile support (2) defines a microporosity and a macroporosity.

9. The part as claimed in the preceding claim, wherein the absorbable material (3) covers the textile support (2) by means of a coating which occludes the microporosity of the textile support.

10. The part as claimed in the preceding claim, wherein the coating also occludes the macroporosity of the textile support (2) in a zone called the occluded zone.

11. The part as claimed in any one of the preceding claims, wherein said part is rectangular, and wherein the protected zone (4) represents a central band of said part.

12. The part as claimed in claim 10, wherein said part is rectangular, and wherein the occluded zone represents a central band of said part.

13. Use of the intermediate composite part as claimed in any one of claims 1 through 12, for obtaining a composite reinforcement prosthesis.

14. Process for obtaining a composite reinforcement prosthesis, wherein:
- i) an intermediate composite part (1) as claimed in any one of claims 1 through 12 is obtained,
- ii) the composite part (1) is cut out along a cutting line defining in this part at least two zones of tissue attachment (10, 14, 18), on each side of a zone of tissue support (9, 13, 17), the attachment zones being cut out from the two unprotected zones (5, 6), respectively, and the tissue support zone being cut out from the protected zone (4).

15. The process as claimed in claim 14, wherein the cutting line defines a strip with straight parallel edges (8).

16. The process as claimed in claim 14, wherein the cutting line defines a strip with parallel edges curved in an arch (12).

17. The process as claimed in claim 14, wherein the cutting line defines a strip with nonparallel edges (16), which is bulged in the central region and narrower at the ends.

## Patentansprüche

1. Kompositzwischenstück (1), das für die Gestaltung einer Kompositverstärkungsprothese angepasst ist, **dadurch gekennzeichnet, dass** es aufweist:
- i) einen porösen textilen Träger (2), der eine Anordnung von Fäden aufweist, die jeweils aus zumindest einer Elementarfaser aus nicht resorbierbarem polymerem Material aufgebaut sind, wobei der textile Träger den oder die freien Ränder (7) des Stückes (1) bestimmt, und
- ii) ein hydrophiles resorbierbares Material (3), das den textilen Träger zumindest auf einer Seite des letzteren bedeckt, und sich hinsichtlich der Oberfläche entlang einer als geschützt bezeichneten Zone (4) erstreckt und beidseits der letzteren zwei als nicht geschützt bezeichnete Zonen (5, 6) des textilen Trägers ausbildet, die von den freien Rändern begrenzt sind und frei von diesem resorbierbaren Material sind.

2. Stück nach Anspruch 1, **dadurch gekennzeichnet, dass** die Anordnung von Fäden eine gewirkte Struktur bildet.

3. Stück nach Anspruch 1 oder 2, **dadurch gekennzeichnet, dass** der textile Träger (2) eine zweidimensionale Struktur bildet.

4. Stück nach einem der vorhergehenden Ansprüche, **dadurch gekennzeichnet, dass** das nicht resorbierbare polymere Material aus der Gruppe ausgewählt ist, die die Polypropylene, Polyester, Polyamide und/oder ihre Gemische umfasst.

5. Stück nach einem der vorhergehenden Ansprüche, **dadurch gekennzeichnet, dass** das resorbierbare Material (3) aus der Gruppe ausgewählt ist, die durch die Kollagene, Polysaccharide und ihre Gemische gebildet ist.

6. Stück nach einem der vorhergehenden Ansprüche, **dadurch gekennzeichnet, dass** das resorbierbare Material (3) den textilen Träger (2) auf seinen beiden Seiten bedeckt.

7. Stück nach Anspruch 1 oder 2, **dadurch gekennzeichnet, dass** das resorbierbare Material (3) den textilen Träger (2) mittels einer Membrane bedeckt, die mit dem textilen Träger durch Kleben und/oder teilweises Imprägnieren verbunden ist.

8. Stück nach Anspruch 1 oder 2, **dadurch gekennzeichnet, dass** der textile Träger (2) eine Mikroporosität und eine Makroporosität definiert.

9. Stück nach dem vorhergehenden Anspruch, **dadurch gekennzeichnet, dass** das resorbierbare Material (3) den textilen Träger (2) mittels eines Auftrages bedeckt, der die Mikroporosität des textilen Trägers verschließt.

10. Stück nach dem vorhergehenden Anspruch, **dadurch gekennzeichnet, dass** der Auftrag auch die Makroporosität des textilen Trägers (2) über eine als verschlossen bezeichnete Zone verschließt.

11. Stück nach einem der vorhergehenden Ansprüche, **dadurch gekennzeichnet, dass** es rechteckig ist und dass die geschützte Zone (4) einen mittigen Streifen des Stückes darstellt.

12. Stück nach Anspruch 10, **dadurch gekennzeichnet, dass** es rechteckig ist und dass die verschlossene Zone einen mittigen Streifen des Stücks darstellt.

13. Verwendung eines Kompositzwischenstücks gemäß einem der Ansprüche 1 bis 12 zum Herstellen einer Kompositverslärkungsprothese.

14. Verfahren zum Herstellen einer Kompositversttirkungsprothese, **dadurch gekennzeichnet, dass**:
- i) ein Kompositzwischenstück (1) gemäß einem der Ansprüche 1 bis 12 bereitgesteilt wird,
- ii) das Kompositstück (1) entlang einer Schnittlinie zugeschnitten wird, die in letzterem zumindest zwei Gewebeankoppelzonen (10, 14, 18) beidseits einer Gewebestülzzone (9, 13, 17) bestimmt, wobei die Ankoppelzonen jeweils in die beiden nicht geschützten Zonen (5, 6) zugeschnitten werden und die Gewebestützzone in die geschützte Zone (4) zugeschnitten wird.

15. Verfahren nach Anspruch 14, **dadurch gekennzeichnet, dass** die Schnittlinie einen kleinen Streifen mit geraden parallelen Rändern (8) bestimmt.

16. Verfahren nach Anspruch 14, **dadurch gekennzeichnet, dass** die Schnittlinie einen kleinen Streifen mit bogenförmig gekrümmten parallelen Rändern bestimmt.

17. Verfahren nach Anspruch 14, **dadurch gekennzeichnet, dass** die Schnittlinie einen kleinen Streifen mit nicht parallelen Rändern (16) bestimmt, der im mittigen Bereich bauchig und an den Enden schmal ist.
